# FASCICULE DE BREVET EUROPEEN

(11) **EP 2 523 631 B1**
(45) Date de publication et mention de la délivrance du brevet: **26.02.2014**
(21) Numéro de dépôt: 10818098.5
(22) Date de dépôt: 22.12.2010
(51) Int. Cl.: A61F 2/16

(54) **INJECTEUR POUR IMPLANT OPHTALMOLOGIQUE PLIABLE**
INJEKTOR FÜR EIN FLEXIBLES AUGENIMPLANTAT
INJECTOR FOR A FLEXIBLE OPHTHALMOLOGIC IMPLANT

(30) Priorité: 12.01.2010 FR 1000096
(43) Date de publication de la demande: 21.11.2012
(73) Titulaire: Mohabeddine, Sadek, 16000 Alger (DZ)
(72) Inventeur: Mohabeddine, Sadek, 16000 Alger (DZ)
(74) Mandataire: Vander-Heym, Serge Henri Marcel
(86) Numéro de dépôt international: PCT/IB2010/003500
(87) Numéro de publication internationale: WO 2011/086418

(56) Documents cités:
- EP-A1- 2 060 243
- US-A1- 2002 193 803
- US-B1- 6 500 181

## Description

La présente invention est relative à un injecteur pour implant ophtalmologique pliable.

Dans le traitement de la cataracte, on élimine le cristallin et on le remplace par une lentille introduite dans la capsule cristalline. Cette lentille est réalisée en une matière souple et élastiquement déformable, pouvant être pliée et comprimée.

En l'état actuel de la technique, l'implant se présente sous la forme d'une lentille biconvexe d'un diamètre de 3 à 7 millimètres qui comporte à sa périphérie des anses se présentant, généralement, sous la forme de bras curvilignes ayant pour fonction de centrer la lentille dans la capsule cristalline l'ensemble ayant un diamètre total de l'ordre 10 à 15 mm.

L'implant est plié et introduit dans une aiguille creuse comportant un poussoir permettant de l'éjecter lorsque la pointe de l'aiguille est introduite dans la capsule cristalline.

On connaît un injecteur dans lequel l'aiguille creuse présente une ouverture latérale débouchant dans une chambre dans laquelle l'implant est disposé, ladite chambre présentant à l'opposé de l'ouverture une palette pivotante.

Le pivotement de la palette a pour effet de replier l'implant et de l'introduire dans le conduit de l'aiguille, ladite palette, en fin de mouvement, fermant l'ouverture précitée. A ce moment, il est possible d'actionner un poussoir qui éjecte l'implant, l'injecteur se comportant à l'instar d'une seringue usuelle.

Ce dispositif ne donne pas entière satisfaction pour plusieurs raisons.

L'expérience a montré qu'il n'était pas possible d'obtenir un résultat satisfaisant avec tous les implants existants dont les anses sont de formes variées.

Il résulte des explications ci-dessus que la mise en oeuvre de l'injecteur nécessite deux manipulations qui doivent être effectuées dans un ordre précis et dont le déroulement dépend de l'opérateur. Ainsi, il importe que le praticien chargé de l'opération actionne la palette et attende la fin de son pivotement avant d'agir sur le poussoir.

Il s'avère donc que l'utilisation de cet injecteur requiert une attention très soutenue de la part du praticien.

Au document US 2002/0193803 il est décrit un injecteur tel que défini au préambule de la revendication 1. Une seule et même tige y a pour fonction de plier et de pousser l'implant.

Le but de l'invention est de proposer un injecteur permettant de plier et d'éjecter un implant par une action continue sur un poussoir.

Cet injecteur est remarquable en ce qu'il est tel que défini à la revendication 1.

La présente invention sera mieux comprise par la description qui va suivre faite en se référant aux dessins annexés à titre d'exemple indicatif seulement sur lesquels :

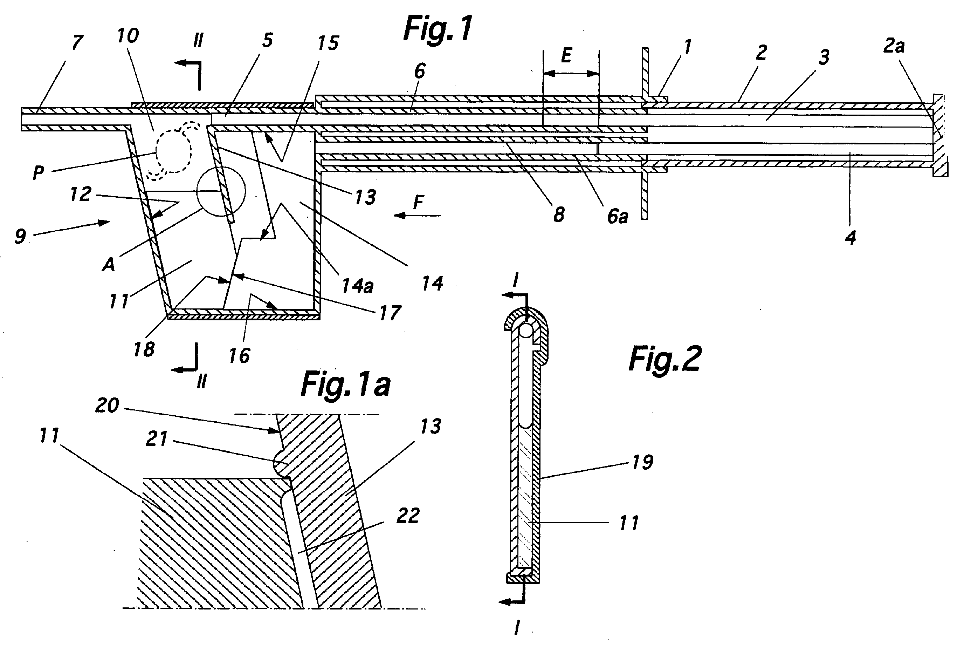

- la figure 1 est une vue en coupe effectuée selon la ligne 1-1 de la figure 2, montrant un injecteur prêt à l'emploi ;
- la figure 1 a est une vue à plus grande échelle du détail A de la figure 1 ;
- la figure 2 est une vue en coupe effectuée selon la ligne II-II de la figure 1 ;
- la figure 3 est une vue analogue à celle 1 montrant la position des éléments mobiles à la fin de l'opération (l'implant ayant été éjecté) ;
- la figure 4 est une vue en coupe effectuée selon la ligne IV-IV de la figure 3 ;
- la figure 4a est une vue à plus grande échelle de la partie supérieure de la figure 4 ;
- la figure 5 est une vue partielle et à plus grande échelle de la partie de l'injecteur comportant le poussoir de pliage ;
- la figure 6 est une vue en coupe effectuée selon la ligne VI-VI de la figure 5, les organes mobiles étant représentés en position initiale ;
- la figure 7 est une vue analogue à celle 6, les organes mobiles étant représentés en position finale ;
- la figure 8 est une vue en perspective du poussoir de pliage.

En se reportant aux dessins, on voit que l'injecteur comporte un corps 1, de guidage d'un organe tubulaire 2, ou poussoir général, renfermant deux tiges parallèles 3 et 4.

La tige 3, par son déplacement, peut agir sur un poussoir d'éjection 5, guidé dans un tube 6 du corps 1 et formant le prolongement de l'aiguille creuse 7 usuelle.

La tige 4, guidée dans un tube 6a peut, par son déplacement, agir sur un poussoir de pliage 8 débouchant dans un boîtier 9 du corps.

Le boîtier 9 présente un compartiment 10, dans lequel est disposé l'implant P, débouchant latéralement dans l'aiguille 7.

Le pliage de l'implant est réalisé par le déplacement d'une pièce de pliage 11, guidée entre les parois parallèles 12 et 13 du boîtier.

Le déplacement de la pièce 11 est assuré par une came 14 guidée entre les parois parallèles 15 et 16 du boîtier.

La came 14 présente une partie oblique 17 en contact avec une seconde partie oblique 18 de l'organe de pliage 11 de sorte que lorsque ladite pièce se déplace selon la flèche F celle-ci oblige ledit organe de pliage à se déplacer vers le haut en repoussant l'implant P qui se replie et se loge dans le tube formé par le boîtier et le bord supérieur de l'organe de pliage.

II ressort des explications ci-dessus que le déplacement d'une pièce, celle 14, selon une direction détermine le déplacement de l'autre, celle 11, selon une autre direction.

A la fin du déplacement, il est alors possible à l'aide du poussoir 5 d'éjecter l'implant dans la capsule cristalline.

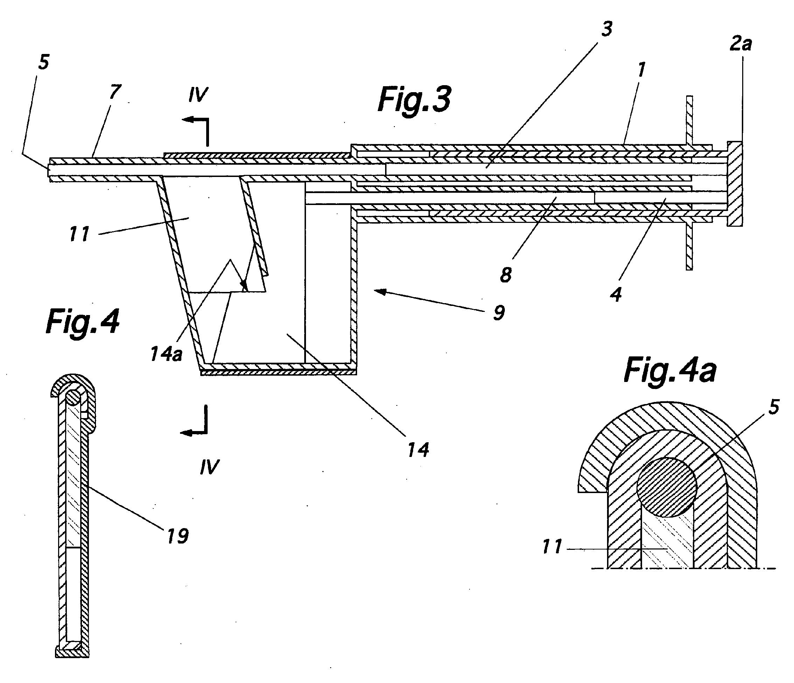

L'expérience a montré que le pliage de l'implant était facilité si l'épaisseur de l'organe de pliage, dont le champ supérieur est conformé pour reproduire le profil du canal de l'aiguille 7, était légèrement inférieure au diamètre dudit canal comme cela ressort de la figure 4a. II en résulte que la partie fixe du canal, de réception de l'implant plié,s'étende sur plus de 180°.

Selon un mode de réalisation, le boîtier 9 présente une face ouverte, permettant la mise en place des organes mobiles 11 et 14 et de l'implant, cette face étant normalement obturée par un couvercle 19, fixé, par exemple par clipsage.

Naturellement des moyens sont prévus pour maintenir l'organe dans la position représentée sur la figure 1, comme cela ressort de la figure 1 a. Ainsi, il est possible de prévoir sur la face 20 de la paroi 13 une petite protubérance 21 contre laquelle prend appui la partie supérieure de l'organe 11. Un effort très léger est suffisant pour franchir cet obstacle lorsque la pièce 11 est repoussée par celle 14. Pour que cet effort complémentaire ne soit pas constant, une rainure 22 est prévue sur le champ correspondant de la pièce 11.

Le déplacement de la came 14 et, par suite, celui de la pièce 11 est placé sous la dépendance du poussoir 8.

Durant le déplacement du poussoir 8 sous l'action de la tige 4, il importe que la tige 3, dont le déplacement est lié à celui de la tige 4, soit sans action sur le poussoir d'éjection 5.

Un tel résultat peut être obtenu en prévoyant, entre la tige 3 et le poussoir 5, un espace initial E, au moins égal à la course utile du poussoir 8. Selon un autre mode de réalisation, la longueur du poussoir est réduite de la valeur de l'espace E précité et l'extrémité antérieure du poussoir est, initialement, en contact avec l'extrémité postérieure de la tige 3. Selon une variante de ce mode de réalisation, le poussoir et sa tige sont réalisés en une seule pièce.

Naturellement des moyens sont prévus pour maintenir le poussoir 5 dans la position représentée sur la figure 1 et s'opposer à son déplacement intempestif. Ce résultat peut être obtenu en prévoyant un jeu relativement ajusté, dit « à frottement gras », entre le poussoir et son alésage. On peut utiliser, aussi un moyen analogue à celui utilisé pour le maintien de la pièce 11.

En agissant sur l'extrémité 2a du tube de support des tiges 3 et 4, on agit sur l'extrémité du poussoir de pliage et l'on repousse la pièce 14 qui force le déplacement de celle 11.

A la fin du mouvement, le talon 14a de la pièce 14 s'insère sous celle 11 et assure le verrouillage de cette dernière.

Des moyens sont prévus pour que, lorsque les pièces 11 et 14 sont dans la position représentée sur la figure 3, il soit possible de poursuivre le déplacement du poussoir général 2.

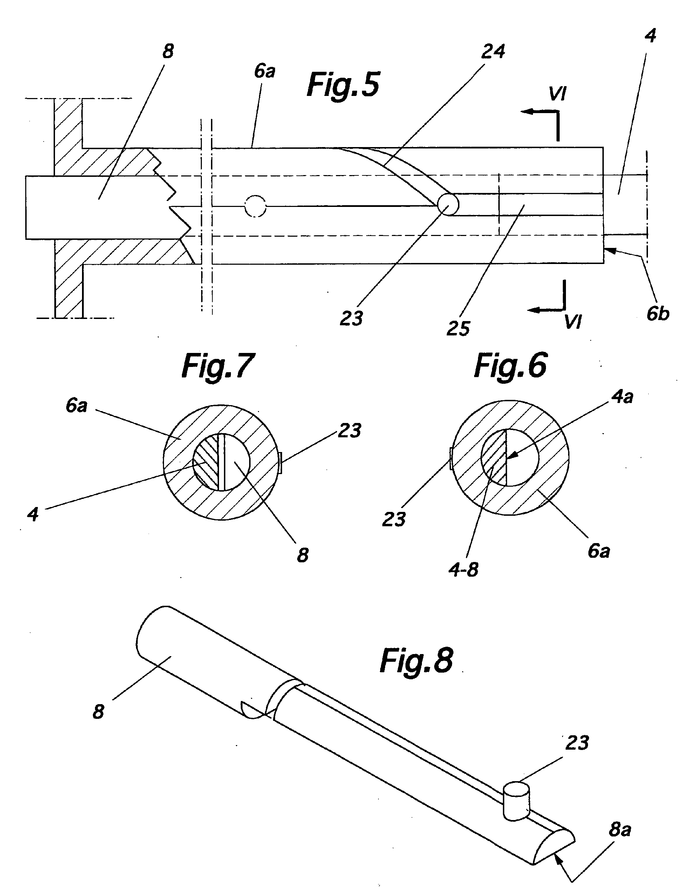

Un tel moyen va être décrit en regard des figures 5 à 8.

Le poussoir 8 présente un méplat 8a très prononcé sur la plus grande partie de sa longueur et la tige 4 présente un méplat identique 4a de sorte que, et comme cela ressort de la figure 7, les deux parties méplates puissent coulisser librement dans le tube guide 6a.

Initialement le poussoir 8 et la tige 4 sont positionnés angulairement de façon à ce que l'extrémité de la tige 4 soit en contact avec celle du poussoir 8, c'est la position qui correspond à la coupe de la figure 6.

Des moyens sont prévus pour qu'à la fin du déplacement axial du poussoir 8 la position angulaire de ce dernier et celle de la tige 4 correspondent à celle montrée sur la coupe de la figure 7, position selon laquelle la tige 4 coulisse librement dans le tube guide 6a alors que le poussoir d'éjection est soumis à l'action de la tige 3.

Le poussoir 8 présente un doigt 23 guidé dans une rainure hélicoïdale 24 du guide 6a. De cette façon, lorsque le poussoir 8 est soumis à l'action de la tige 4, il pivote pour se trouver, à la fin du mouvement, dans la position représentée sur la coupe de la figure 7. La rainure s'étend sur 180° et son pas est égal à la moitié du déplacement longitudinal du poussoir 8.

Pour un problème de montage, la rainure 24 débouche dans une rainure longitudinale 25.

Le doigt 23 peut avantageusement être utilisé pour servir d'appui à un ressort hélicoïdal, à spires non jointives, monté soit sur le tube 6a , soit sur celui 6. Le ressort est comprimé lorsque l'action du poussoir général est efficace et il se détend en provoquant un recul du poussoir correspondant lorsque la tige correspondante n'a plus d'effet sur le poussoir associé.

## Revendications

1. Injecteur pour implant ophtalmologique pliable comportant un poussoir général (2) présentant deux tiges (3-4) dont l'une (3) peut agir sur un poussoir d'éjection (5), **caractérisé en ce que** l'autre tige (4) peut agir, par l'entremise d'un poussoir de pliage (8), sur un mécanisme permettant le pliage de l'implant (P) et son introduction dans une aiguille creuse (7), des moyens étant prévus pour inhiber, lorsque cela est souhaitable, l'action de l'une ou l'autre des tiges du poussoir.

2. Injecteur suivant la revendication 1, caractérisé le mécanisme de pliage de l'implant se compose de deux pièces (11 et 14) dont le déplacement (F) de l'une sous l'action du poussoir de pliage détermine le déplacement de l'autre, selon une autre direction.

3. Injecteur selon la revendication 2, **caractérisé en ce que** l'épaisseur de la pièce de pliage (11) est légèrement inférieure au diamètre du canal de l'aiguille (7).

4. Injecteur selon l'une des revendications 2 et 3, **caractérisé en ce qu'**il comporte des moyens pour s'opposer au déplacement intempestif de la pièce de pliage (11).

5. Injecteur selon l'une des revendications 1 à 4, **caractérisé en ce qu'**un espace initial (E), au moins égal à la valeur du déplacement du poussoir de pliage (8), est ménagé entre l'extrémité du poussoir d'éjection (5) et celle de la tige correspondante (3).

6. Injecteur selon la revendication 5, **caractérisé en ce que** la longueur du poussoir (8) est réduite de la valeur de l'espace (E) et **en ce que** l'extrémité antérieure du poussoir est, initialement, en contact avec l'extrémité postérieure de la tige (3).

7. Injecteur selon la revendication 6, **caractérisé en ce que** le poussoir (8) et la tige (3) sont réalisés en une seule pièce.

8. Injecteur selon l'une des revendications 1 à 7, **caractérisé en ce que** le poussoir de pliage (8) et sa tige de commande (4) présentent chacun une partie méplate (8a-4a) permettant aux deux organes de coulisser librement dans un tube guide (6a).

9. Injecteur selon l'une des revendications 1 à 8, **caractérisé en ce que** des moyens sont prévus pour obtenir la rotation angulaire du poussoir de pliage durant le déplacement axial de celui-ci.

10. Injecteur selon la revendication 9, **caractérisé en ce que** le poussoir de pliage présente un doigt (23) guidé dans une rainure hélicoïdale (24).

## Patentansprüche

1. Injektor für ein faltbares Augenimplantat, umfassend einen Hauptstößel (2), der zwei Stangen (3-4) umfasst, von denen die eine (3) auf einen Ausdrückstößel (5) wirken kann, **dadurch gekennzeichnet, dass** die andere Stange (4) über einen Faltstößel (8) auf einen Mechanismus wirken kann, der das Falten des Implantats (P) und sein Einführen in eine Hohlnadel (7) ermöglicht, wobei Mittel vorgesehen sind, um das Wirken der einen oder der anderen Stange des Stößels zu hemmen, wenn dies wünschenswert ist.

2. Injektor nach Anspruch 1, **dadurch gekennzeichnet, dass** der Mechanismus zum Falten des Implantats aus zwei Teilen (11 und 14) besteht, wobei die Bewegung (F) des einen unter der Wirkung des Faltstößels die Bewegung des anderen in eine andere Richtung bewirkt.

3. Injektor nach Anspruch 2, **dadurch gekennzeichnet, dass** die Dicke des Faltteils (11) geringfügig kleiner als der Durchmesser des Kanals der Nadel (7) ist.

4. Injektor nach einem der Ansprüche 2 und 3, **dadurch gekennzeichnet, dass** er Mittel umfasst, um sich der unbeabsichtigten Bewegung des Faltteils (11) zu widersetzen.

5. Injektor nach einem der Ansprüche 1 bis 4, **dadurch gekennzeichnet, dass** ein Anfangsabstand (E) von mindestens gleich dem Wert der Bewegung des Faltstößels (8) zwischen dem Ende des Ausdrückstößels (5) und dem der entsprechenden Stange (3) angeordnet ist.

6. Injektor nach Anspruch 5, **dadurch gekennzeichnet, dass** die Länge des Stößels (8) um den Wert des Abstands (E) vermindert ist und dass das vordere Ende des Stößels zu Beginn das hintere Ende der Stange (3) berührt.

7. Injektor nach Anspruch 6, **dadurch gekennzeichnet, dass** der Stößel (8) und die Stange (3) einteilig ausgeführt sind.

8. Injektor nach einem der Ansprüche 1 bis 7, **dadurch gekennzeichnet, dass** der Faltstößel (8) und seine Betätigungsstange (4) jeweils einen flachen Abschnitt (8a-4a) aufweisen, der es den beiden Organen ermöglicht, frei in einer Führungshülse (6a) zu gleiten.

9. Injektor nach einem der Ansprüche 1 bis 8, **dadurch gekennzeichnet, dass** Mittel vorgesehen sind, um die Winkeldrehung des Faltstößels während seiner axialen Bewegung zu erhalten.

10. Injektor nach Anspruch 9, **dadurch gekennzeichnet, dass** der Faltstößel einen Finger (23) aufweist, der in einer Wendelnut (24) geführt wird.

## Claims

1. Injector for a bendable ophthalmological implant, comprising a general thruster (2) with two rods (3-4), one of which can act on an ejection thruster (5), **characterised in that** the other rod (4) can act, by means of the interposition of a bending thruster (8), on a mechanism which permits bending of the implant (P) and its introduction into a hollow needle (7), means being provided in order to inhibit the action of one or the other of the rods of the thruster when this is desirable.

2. Injector according to claim 1, **characterised in that** the mechanism for bending the implant consists of two parts (11 and 14), the displacement of one of which under the action of the bending thruster gives rise to the displacement of the other, in another direction.

3. Injector according to claim 2, **characterised in that** the thickness of the bending part (11) is slightly smaller than the diameter of the channel of the needle (7).

4. Injector according to one of claims 2 and 3, **characterised in that** it comprises means for opposing the untimely displacement of the bending part (11).

5. Injector according to one of claims 1 to 4, **characterised in that** an initial space (E), which is at least equal to the value of the displacement of the bending thruster (8), is provided between the end of the ejection thruster (5) and that of the corresponding rod (3).

6. Injector according to claim 5, **characterised in that** the length of the thruster (8) is reduced of the value of the space (E), and **in that** the front end of the thruster is initially in contact with the rear end of the rod (3).

7. Injector according to claim 6, **characterised in that** the thruster (8) and the rod (3) are made in a single piece.

8. Injector according to one of claims 1 to 7, **characterised in that** the bending thruster (8) and its control rod (4) each have a flattened part (8a-4a) which allows the two units to slide freely in a guide tube (6a).

9. Injector according to one of claims 1 to 8, **characterised in that** means are provided in order to obtain the angular rotation of the bending thruster during the axial displacement of the latter.

10. Injector according to claim 9, **characterised in that** the bending thruster has a finger (23) which is guided in a helical groove (24).
